# EUROPEAN PATENT APPLICATION

(11) **EP 4 008 712 A1**
(43) Date of publication of application: **08.06.2022**
(21) Application number: 20848133.3
(22) Date of filing: 08.06.2020
(51) Int. Cl.: C07C 227/36, C07C 231/12, C07D 241/08, C07D 295/185, C07D 471/04, C07D 487/04, C07C 229/34, C07C 233/47, C07C 229/12, C07C 229/18, C07B 55/00

(54) **RACEMIC PREPARATION METHOD FOR CHIRAL BETA-AMINO ACID AND DERIVATIVE THEREOF**

(30) Priority: 01.08.2019 CN 201910705432
(71) Applicant: Zhejiang Jiuzhou Pharmaceutical Co., Ltd, Taizhou City, Zhejiang 318000 (CN)
(72) Inventor: LI, Hongliang, Zhejiang 318000 (CN); ZHANG, Xianyi, Zhejiang 318000 (CN); GAO, Zhaobo, Zhejiang 318000 (CN); MEI, Yijiang, Zhejiang 318000 (CN)
(74) Representative: IPrime Bonnekamp Sparing Patentanwaltsgesellschaft mbH
(86) International application number: PCT/CN2020/094818
(87) International publication number: WO 2021/017645

(57) **Abstract**

The present disclosure relates to the field of medicine synthesis, and relates to a method for a racemic preparation of chiral β -amino acids and derivatives thereof, and in particular to a method for racemizing sitagliptin intermediates. 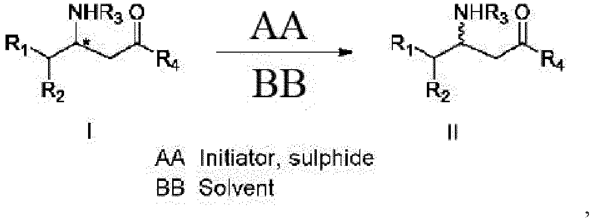 where R₁ is hydrogen, alkyl or aryl; R₂ is hydrogen, alkyl or aryl; R₃ is hydrogen, alkyl, aryl or acyl; R₄ is alkyl, aryl, OR', SR', NHR' or NR'R", wherein R', R" are hydrogen, alkyl or aryl.

## Description

### CROSS-REFERENCE TO RELATED APPLICATION

This application claims priority to Chinese Patent Application No. 201910705432.5, dated August 1, 2020 and entitled "METHOD FOR RACEMIC PREPARATION OF CHIRAL β-AMINO ACIDS AND DERIVATIVES THEREOF", the disclosure of which is herein incorporated by reference in its entirety.

### TECHNICAL FIELD

The present disclosure relates to the field of medical synthesis, and relates to a method for a racemic preparation of chiral *β* -amino acids and derivatives thereof, in particular to a method for a racemic recycling of sitagliptin and intermediates thereof.

### BACKGROUND

Sitagliptin, with a chemical name (2R)-4-oxo-4-[3-trifluoromethyl-5,6-dihydro[1,2,4]triazole[4,3-a]pyrazine-7(8H)-yl]-1-(2,4,5-trifluorophenyl)-butan-2-amine, the structural formula is list as below:

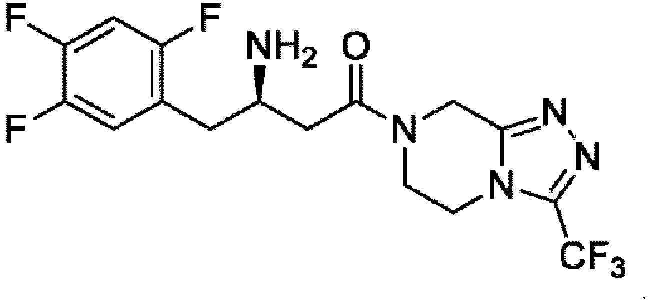

It is the first product of dipeptidyl peptidase-IV (DPP-IV) developed by Merck (US). In October 2006, its phosphate hydrate was approved by the US FDA as the first dipeptidyl peptidase-IV (DPP-IV) for the clinical treatment for clinically treating type 2 diabetes. The medicine has the advantages of fewer adverse reactions, low risk of hypoglycemia and no weight gain.

The key to the synthesis of sitagliptin lies in the construction of the chiral amino group in its *β* -amino acid structure. The current common methods for constructing this type of structure include the asymmetric hydrogenation method and chiral induction method, but this method has problems such as difficulty in preparing catalysts and ligands, and high cost. In addition, the catalytic synthesis method is also an important method for the construction of chiral *β* -amino acid, but this method has problems such as complex equipment required and poor stability, which also limits its industrial application to a certain extent. Therefore, the conventional chiral resolution method is still the mainstream method for the industrial preparation of chiral *β* - amino structure compounds, but most of the remaining enantiomers cannot be effectively recovered, resulting in problems such as high three-waste, high production costs and waste of resources and the like. Therefore, how to solve the problem of the racemization of the chiral *β* -amino group is the key to the industrial production of sitagliptin.

In 2010, Satyanarayana et al. (WO2010122578) reported that the key *β* - amino intermediate of sitagliptin was resolved and the remaining S-isomer III reacted with sodium nitrite and sulfuric acid to undergo a recrystallization reaction and converted into alcohol IV. The recovery of the racemic I can be obtained by oxidizing, aminating, reduction. The method has long steps, complicated operations, and serious environmental pollution, poor industrial feasibility.

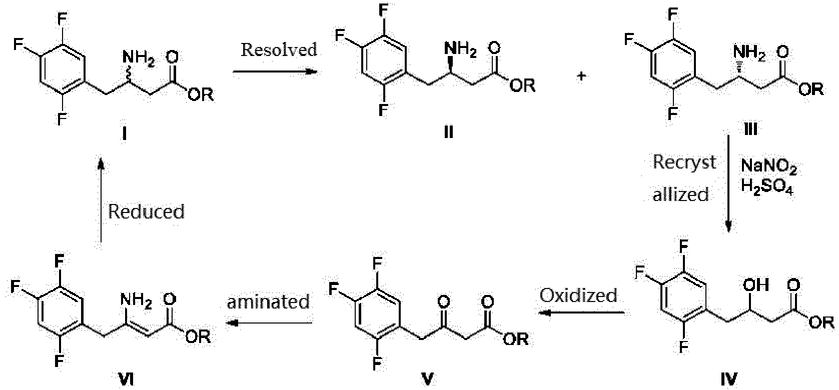

In 2011, Lipeng Zhang et al. (CN102319142) reported that the remaining S-isomer III after the resolution was condensed with aromatic aldehydes to synthesize imine II, followed by palladium-catalyzed hydrogenation and debenzylation to recover the racemic *β* -amino isomer I. This method still has long steps, the yield is low, and expensive heavy metal palladium is needed, which is expensive, so it is difficult to realize industrial production.

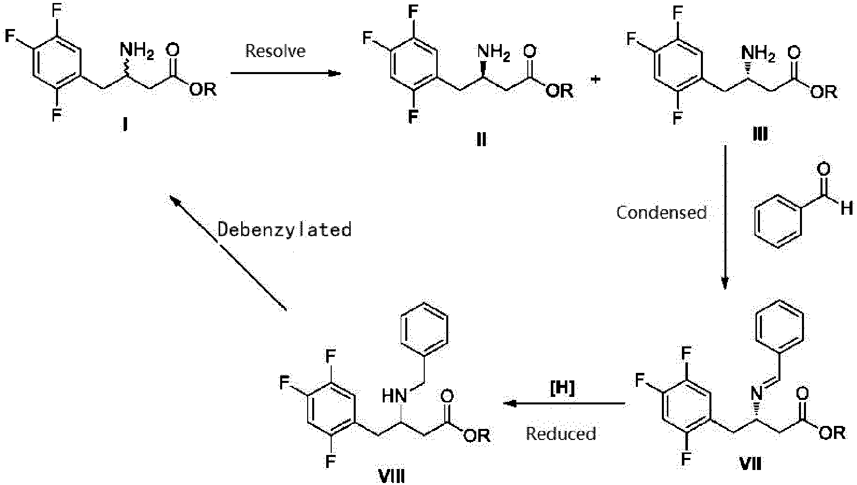

In 2014, Jiansheng Xia et al. (CN04130149) reported that the amino group in the S-isomer was chlorinated to obtain an intermediate IX, and then one molecule of hydrogen chloride was eliminated under alkaline conditions to obtain enamine intermediate VI, which was then reduced by hydrogenation to obtain *β* -amino racemate I. The method has long steps, a large amount of chlorinated reagents and a large amount of alkali are required. Operation is complicated, and high environmental protection pressure exists. Therefore, it is difficult to industrialize feasibility.

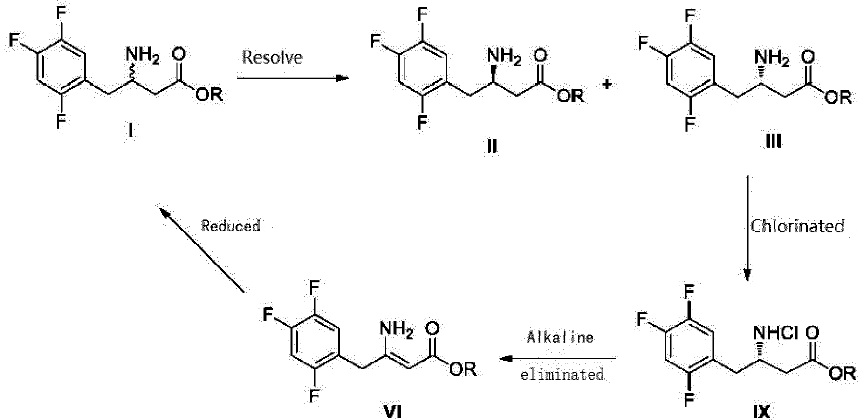

Journal of Organic Chemistry, 71(19), 7288-7292; 2006 discloses that (S)-4-phenyl-2-butylamine was refluxed for 2 hours under the conditions of methyl thioglycolate, benzene and AIBN to obtain 4-phenyl-2-butylamine:

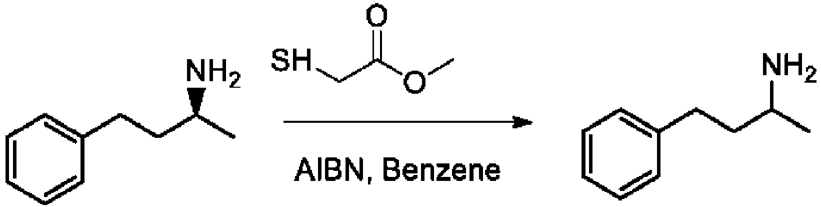

This method can realize the racemization of the chiral amino group in one step, but it also has certain shortcomings: 1) It needs to use the equivalent sulfide, which has a large taste, serious pollution and high cost; 2) It needs to use 0.6 times the equivalent of the initiator AIBN, and needs to be fed in batches, the operation is complicated and cumbersome; 3) There is no report on the racemization of *β* -amino acid and derivatives thereof in this method.

In view of the importance of the method for a racemic preparation of chiral *β* -amino acid and derivatives thereof in the synthesis of sitagliptin intermediates, it is necessary to research and develop a new process to solve the current problems. The process needs to have the advantages such as convenient raw materials and easy operation, low cost, environmentally friendly, and economic and efficiency etc.. Based on this, the present disclosure has developed a method for a racemic preparation of *β* -amino acid and derivatives thereof, in which the amount of sulfide and initiator can be reduced to 1% at most, and the initiator can be fed at one time, which is convenient to operate and environmentally friendly. It can be adapted to industrial production. The specific reaction is as follows:

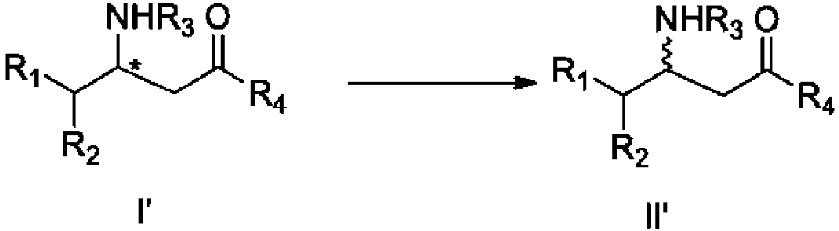

Chiral *β* -amino acids and derivatives thereof cause sulfides to form sulfur free radicals in the presence of initiators, and the sulfur free radicals extract hydrogen from the carbon atom connected to the amino group, thereby realizing the racemization of the amino group.

### SUMMARY

Embodiments of the present disclosure provide a racemization manufacturing method for a racemic preparation of chiral *β* -amino acids of sitagliptin intermediate and derivatives thereof. The raw materials are easy to obtain, the cost is low, and the yield is high, and the method is suitable for industrialized production.

In order to achieve the objects of the present disclosure, the present disclosure provides the following technical solutions:

In a first aspect, the present disclosure provides a method for a racemic preparation of chiral *β* -amino acids and derivatives thereof, comprising the steps of:

racemizing a compound of formula I' under conditions of initiator, sulfide and solvent to obtain a compound of formula II', wherein a reaction equation is: 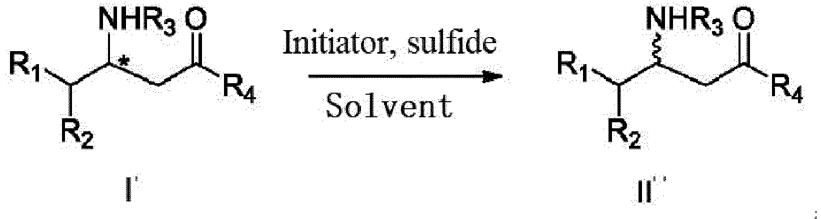
where R₁ is hydrogen, alkyl or aryl; R₁ may preferably be trifluorophenyl.
R₂ is hydrogen, alkyl or aryl; R₂ may preferably be hydrogen.
R₃ is hydrogen, alkyl, aryl or acyl; R₃ may preferably be hydrogen.
R₄ is alkyl, aryl, OR', SR', NHR' or NR'R", where R' or R" is hydrogen, alkyl or aryl. R₄ may preferably be ethoxy, tert-butoxy or isopropoxy.

The sulfide comprises, but not limited to, methyl mercaptoacetate, methyl mercaptopropionate, methyl mercaptobutyrate, ethyl mercaptoacetate, 1-hexyl mercaptan, 1-heptane mercaptan, 1-octyl mercaptan, 1-nonane mercaptan, 1-decane mercaptan, benzyl mercaptan, phenyl ethyl mercaptan, furfuryl mercaptan, dodecane mercaptan and other alkyl mercaptans; 1,6-hexanedithiol, 3-propanedithiol, dimercaptoethyl sulfide, pentaerythritol tetra-3-mercaptopropionate (PETMP), trimethylolpropane tris(3-mercaptopropionate) (TMMP) and other polythiols; mercaptopropyltrimethoxysilane, 3-mercaptopropyltriethoxysilane and other mercapto-containing compounds; dialkyl disulfide, thiophenol, substituted thiophenol and various aromatic thiophenol, diphenyl disulfide and diaryl disulfide, preferably pentaerythritol tetra-3-mercaptopropionate (PETMP) or trimethylolpropane tris(3-mercaptopropionate) (TMMP).

The solvent comprises, but not limited to, one or more from a group consisting of benzene, toluene, o-xylene, m-xylene, p-xylene, nitrobenzene, chlorobenzene, tetrahydrofuran, 2-methyltetrahydrofuran, 1,4-dioxane, anisole, acetonitrile, chloroform, carbon tetrachloride, n-heptane, and n-octane, preferably toluene (TOL) or xylene.

The initiator comprises, but not limited to, azobisisobutyronitrile (AIBN), azobisisovaleronitrile (AMBN), azobisisoheptonitrile (ABVN), azobisisobutamidine hydrochloride (AIBA), dimethyl azobisisobutyrate (V601, AIBME), azobisisobuimidazoline hydrochloride (AIBI), azo isobutylcyanide formamide and other azo initiator (V30); dibenzoyl peroxide (BPO), lauryl peroxide (LPO), Diisopropyl peroxydicarbonate (IPP), dicyclohexyl peroxydicarbonate (DCPD), di-tert-butyl peroxide (DTB), m-chloroperoxybenzoic acid (m-CPBA), potassium persulfate (K₂S₂O₈) and other peroxide initiators, preferably azobisisobutyronitrile (AIBN), azobisisovaleronitrile (AMBN) and azobisisoheptonitrile (ABVN).

A reaction temperature is 30°C to 120°C, preferably 60°C to 100°C.

A reaction time is 4 to 36 hours, preferably 18 to 24 hours.

A molar ratio of the initiator, the sulfide and a chiral *β* -amino substrate required for the reaction is that: initiator: sulfide: substrate is (0.01~0.20): (0.01~0.20): 1.

In a second aspect, the present disclosure provides a method for a racemic preparation of chiral *β* -amino acids and derivatives thereof, comprising the steps of:

racemizing a compound of formula I" under conditions of initiator, sulfide and solvent to obtain a compound of formula II", wherein a reaction equation is:

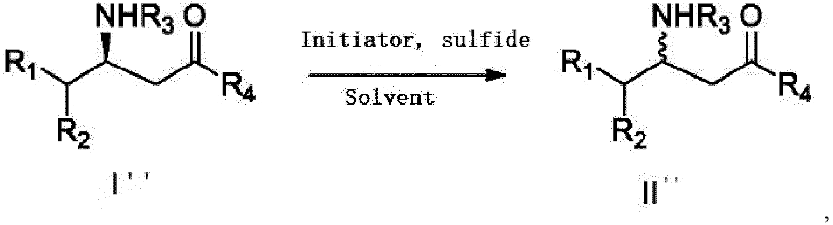

where definitions of R₁, R₂, R₃, R₄ are the same as that above mentioned.

The definition of the sulfide is the same as that above mentioned.

The definitions of the solvent is the same as that above mentioned.

The definitions of the initiator is the same as that above mentioned.

The definitions of the reaction temperature is the same as that above mentioned.

The definitions of the reaction time is the same as that above mentioned.

A molar ratio of the initiator, the sulfide and a chiral *β* -amino substrate required for the reaction is that: initiator: sulfide: substrate is (0.01~0.20): (0.01~0.20): 1.

In a third aspect, the present disclosure provides a method for a racemic preparation of chiral *β* -amino acids and derivatives thereof, comprising the steps of:
racemizing a compound of formula I‴ under conditions of initiator, sulfide and solvent to obtain a compound of formula II", wherein a reaction equation is: 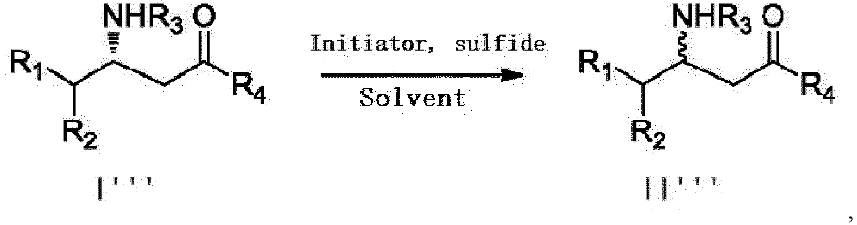 where definitions of R₁, R₂, R₃, R₄ are the same as that above mentioned.

The definition of the sulfide is the same as that above mentioned.

The definitions of the solvent is the same as that above mentioned.

The definitions of the initiator is the same as that above mentioned.

The definitions of the reaction temperature is the same as that above mentioned.

The definitions of the reaction time is the same as that above mentioned.

A molar ratio of the initiator, the sulfide and a chiral *β* -amino substrate required for the reaction is that: initiator: sulfide: substrate is (0.01~0.20): (0.01~0.20): 1.

In a fourth aspect, the present disclosure provides a method for racemizing ethyl 3-amino-4-(2,4,5-trifluorophenyl) butyrate, obtained by racemizing (S)-3-amino-4-(2,4,5-ethyl trifluorophenyl) butyrate under conditions of pentaerythritol tetra-3-mercaptopropionate (PETMP), azobisisobutyronitrile (AIBN) and toluene; wherein a reaction equation is:

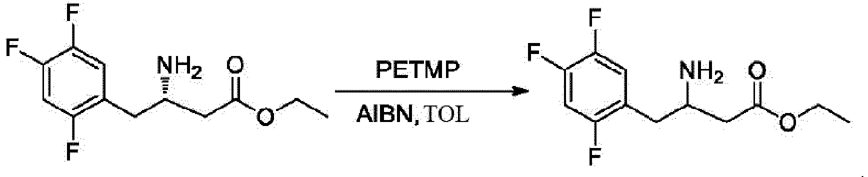

The definitions of the reaction temperature is the same as that above mentioned.

The definitions of the reaction time is the same as that above mentioned.

A molar ratio of the initiator, the sulfide and a chiral *β* -amino substrate required for the reaction is that: initiator: sulfide: substrate is (0.01~0.20): (0.01~0.20): 1.

The present disclosure provides a method for a racemic preparation of chiral *β* -amino acids of sitagliptin intermediate and derivatives thereof, the raw materials are convenient and easy to obtain, with low cost and high yield, the method is suitable for industrial production and preparation. Therefore, the technical solution provided by the present disclosure has high application value in industry.

### DETAILED DESCRIPTION

In order to better understand the content of the present disclosure, further description will be given below in conjunction with specific embodiments, but the specific implementation is not a limitation on the content of the present disclosure.

Example 1:

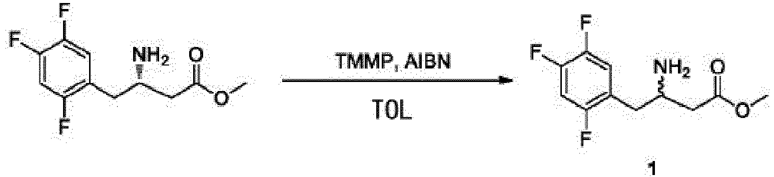

(S)-3-amino-4-(2,4,5-trifluorophenyl) methyl butyrate (4.94g, 0.020mol, ee>99%), toluene (20ml), trimethylolpropane tris(3-mercaptopropionate) (TMMP, 160mg, 0.40mmol) and azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (4.81g, 0.0195 mol), which is the target product compound 1, with a yield of 97.4%; ee=0.78%. MS(ESI): m/z 248.0817[M+H]⁺.

Example 2:

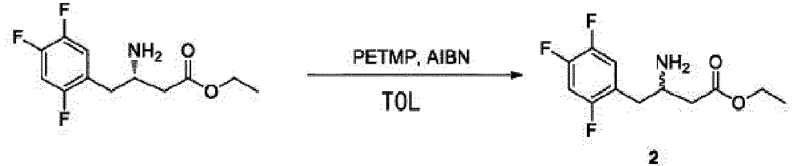

(S)-3-amino-4-(2,4,5-trifluorophenyl) ethyl butyrate (5.22g, 0.020mol, ee>99%), toluene (20ml), pentaerythritol tetra-3-mercaptopropionate (PETMP, 98mg, 0.20mmol) and azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (4.88g, 0.0187mol), which is the target product compound 2, with a yield of 93.5%; ee=0.65%. MS(ESI): m/z 262.0972[M+H]⁺.

Example 3:

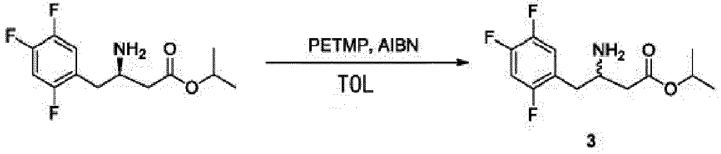

(S)-3-amino-4-(2,4,5-trifluorophenyl) isopropyl butyrate (5.51g, 0.020mol, ee>99%), toluene (20ml), pentaerythritol tetra-3-mercaptopropionate (PETMP, 98mg, 0.20mmol) and azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (5.07g, 0.0184mol), which is the target product compound 3, with a yield of 92%; ee=1.2%. MS(ESI): m/z 276.1128[M+H]⁺.

Example 4:

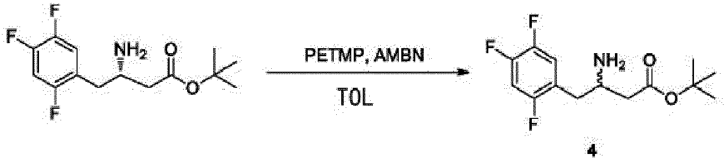

(S)-3-amino-4-(2,4,5-trifluorophenyl) tert-butyl butyrate (5.79g, 0.020mol, ee>99%), toluene (30ml), pentaerythritol tetra-3-mercaptopropionate (PETMP, 98mg, 0.20mmol) and azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 20h. 20 mL of water is added to the reaction solution, the pH value is adjusted to 5~6 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (5.18g, 0.0179mol), which is the target product compound 4, with a yield of 89.5%; ee=1.9%. MS(ESI): m/z 290.1293[M+H]⁺.

Example 5:

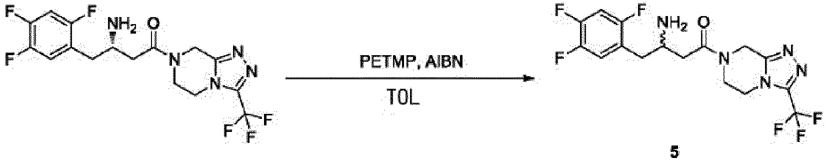

(S)-3-amino-1-(3-(trifluoromethyl)-5,6-dihydro-[1,2,4]triazolone[4,3-a]pyrazine-7(8H)-4-(2,4,5-trifluorophenyl)butanone ((S)-sitagliptin, 8.15g, 0.020mol, ee>99%), toluene (30ml), pentaerythritol tetra-3-mercaptopropionate (PETMP, 98mg, 0.20mmol) and azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 22h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a white solid (7.13g, 0.0175mol), which is the target product compound 5, with a yield of 87.5%; ee=4%. MS(ESI): m/z 408.1179[M+H]⁺.

Example 6:

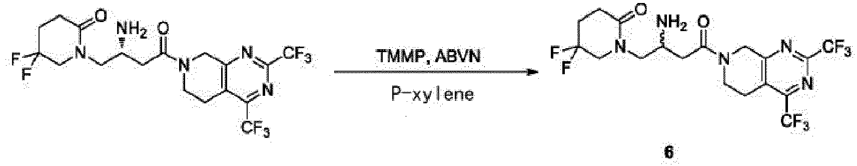

(R)-1-(2-amino-4-(2,4-bis(trifluoromethyl)-5,8-dihydropyrido[3,4-D]pyrimidin-7(6H)-yl) -4-oxobutyl)-5,5-difluoropiperidin-2-one ((R)-gigliptin, 9.79g, 0.020mol, ee>99%), p-xylene (30ml), trimethylolpropane tris(3-mercaptopropionate) (TMMP, 160mg, 0.40mmol) and azobisisoheptonitrile (ABVN, 50mg, 0.20mmol) are put into a 100 ml of three-necked flask, magnetic stirring is turned on. Under nitrogen protection, the temperature rises slowly to 70°C, the progress of the reaction is tracked by HPLC, and the reaction is complete after 22h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with ethyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (8.27g, 0.0169mol), which is the target product compound 6, with a yield of 84.5%; ee=1.6%. MS(ESI): m/z 490.1408[M+H]⁺.

Example 7:

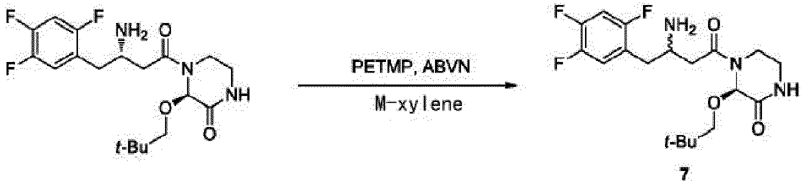

(S)-4((S)-3-amino-4-(2,4,5-trifluorophenyl)butyryl)-3-(tert-butyl methyl ether methyl) piperazin-2-one ((S)-itagliptin, 7.99g, 0.020mol, ee>99%), m-xylene (30ml), pentaerythritol tetra-3-mercaptopropionate (PETMP, 98mg, 0.20mmol) and azobisisoheptonitrile (ABVN, 50mg, 0.20mmol) are put into a 100 ml of three-necked flask, magnetic stirring is turned on. Under nitrogen protection, the temperature rises slowly to 70°C, the progress of the reaction is tracked by HPLC, and the reaction is complete after 24h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with ethyl acetate (2 × 20ml). The organic phases is combined, washed once with saturated brine (20ml), dried with anhydrous sodium sulfate (10g), filtered, and the filtrate is concentrated to dryness under reduced pressure at 35~40 °C to obtain a colorless oil (6.78g, 0.0170mol), which is the target product compound 7, with a yield of 85%; ee=2.2%. MS(ESI): m/z 400.2137[M+H]⁺.

Example 8:

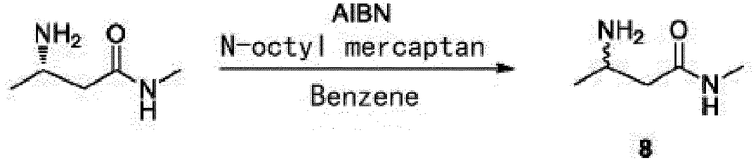

(S)-3-amino-N-methyl-4-butanamide (2.32g, 0.020mol, ee>99%), dry benzene (20ml), n-octyl mercaptan (0.29g, 0.002mol), azobisisobutyronitrile (AIBN, 66mg, 0.40mmol) are put into a 100 ml of three-necked flask, magnetic stirring is turned on. Under nitrogen protection, the temperature rises slowly to 60°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with ethyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (2.21g, 0.0191mol), which is the target product compound 8, with a yield of 95.3%; ee=2.4%. MS(ESI): m/z 117.0944[M+H]⁺.

Example 9:

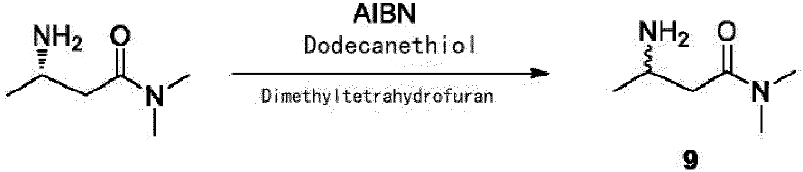

(S)-3-amino-N,N-dimethyl-4-butanamide (2.60g, 0.020mol, ee>99%), dimethyltetrahydrofuran (20ml), dodecanethiol (0.41g, 0.002 mol), azobisisobutyronitrile (AIBN, 66mg, 0.40mmol) are put into a 100 ml of three-necked flask, magnetic stirring is turned on. Under nitrogen protection, the temperature rises slowly to 60°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with ethyl acetate (2 × 20ml). The organic phases is combined, washed once with saturated brine (20ml), dried with anhydrous sodium sulfate (10g), filtered, and the filtrate is concentrated to dryness under reduced pressure at 35~40 °C to obtain a colorless oil (2.43g, 0.0187mol), which is the target product compound 9, with a yield of 95.3%; ee=2.7%. MS(ESI): m/z 131.1102[M+H]⁺.

Example 10:

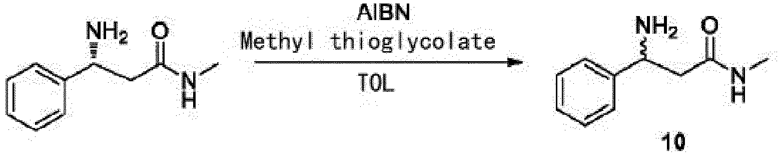

(R)-3-amino-3-phenyl-N-methyl-butyramide (3.56g, 0.020mol, ee>99%), toluene (20ml), methyl thioglycolate (0.42g, 0.004mol), azobisisobutyronitrile (AIBN, 66mg, 0.40mmol) are put into a 100 ml of three-necked flask, magnetic stirring is turned on. Under nitrogen protection, the temperature rises slowly to 80 °C, the progress of the reaction is tracked by HPLC, and the reaction is complete after 22h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (3.25g, 0.0183mol), which is the target product compound 10, with a yield of 91.2%; ee=2.8%. MS(ESI): m/z 179.1114[M+H]⁺.

Example 11:

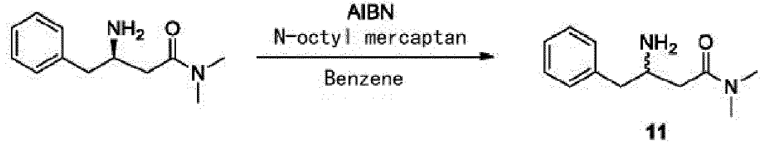

(R)-3-amino-4-phenyl-N,N-dimethylbutanamide (4.12g, 0.020mol, ee>99%), benzene (20ml), n-octyl mercaptan (0.29g, 0.002mol ), azobisisobutyronitrile (AIBN, 66mg, 0.40mmol) are put into a 100 ml of three-necked flask, magnetic stirring is turned on. Under nitrogen protection, the temperature rises slowly to 80 °C, the progress of the reaction is tracked by HPLC, and the reaction is complete after 22h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (3.61g, 0.0175mol), which is the target product compound 11, with a yield of 87.6%; ee=2.6%. MS(ESI): m/z 207.1413 [M+H]⁺.

Example 12:

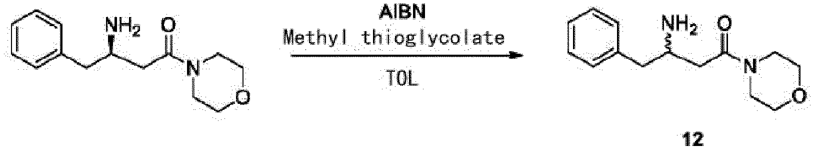

(R)-3-amino-4-phenyl-1-piperidinyl butanone (4.97g, 0.020mol, ee>99%), toluene (20ml), methyl thioglycolate (0.42g, 4.0mmol), azobisisobutyronitrile (AIBN, 66mg, 0.4mmol) are put into a 100 ml of three-necked flask, magnetic stirring is turned on. Under nitrogen protection, the temperature rises slowly to 80 °C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (4.23g, 0.0170mol), which is the target product compound 12, with a yield of 85.1%; ee=2.3%. MS(ESI): m/z 249.1517[M+H]⁺.

Example 13:

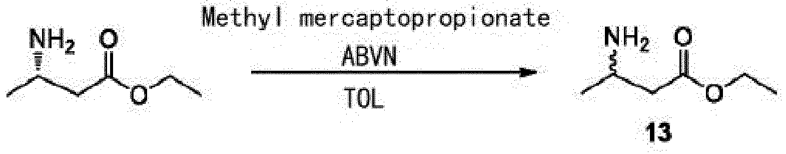

(S)-3-ethyl aminobutyrate (2.62g, 0.020mol, ee>99%), toluene (20ml), methyl mercaptopropionate (0.49g, 4.0mmol), azobisisoheptanitrile (ABVN, 99mg, 0.40mmol) are put into a 100 ml of three-necked flask, magnetic stirring is turned on. Under nitrogen protection, the temperature rises slowly to 60°C, the progress of the reaction is tracked by HPLC, and the reaction is complete after 26h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (2.53g, 0.0193mol), which is the target product compound 13, with a yield of 96.6%; ee=0.8%. MS(ESI): m/z 132.0951 [M+H]⁺.

Example 14:

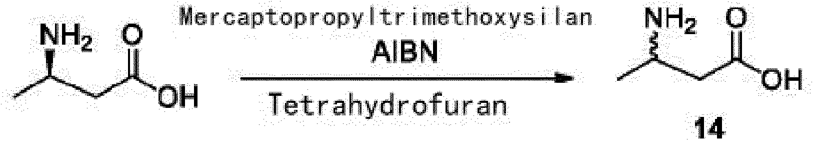

(R)-3-aminobutyric acid (2.06g, 0.020mol, ee>99%), tetrahydrofuran (20ml), water (2mL), mercaptopropyltrimethoxysilane (0.79g, 4.0mmol), azobis isobutyronitrile (AIBN, 66mg, 0.40mmol) are put into a 100 ml of three-necked flask, magnetic stirring is turned on. Under nitrogen protection, the temperature rises slowly to 60°C, the progress of the reaction is tracked by HPLC, and the reaction is complete after 24h. 20 mL of water is added to the reaction solution, the pH value is adjusted to 2~3 with 2N dilute hydrochloric acid, the liquids are separated, the organic phase is discarded. The pH value of the water phase is adjusted to about 4.5with 2N sodium hydroxide solution, a solid is precipitated, filtered to obtain a white viscous solid (1.55g, 0.0150mol), which is the target product compound 14, with a yield of 75.2%; ee=5.9%. MS(ESI): m/z 104.0631[M+H]⁺.

Example 15:

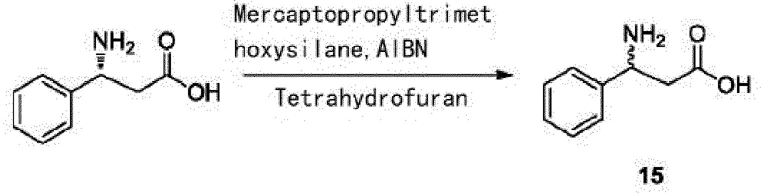

(R)-3-amino-3-phenylpropionic acid (3.31g, 0.020mol, ee>99%), tetrahydrofuran (20ml), mercaptopropyltrimethoxysilane (0.79g, 4.0mmol), azobis isobutyronitrile (AIBN, 66mg, 0.40mmol) are put into a 100 ml of three-necked flask, magnetic stirring is turned on. Under nitrogen protection, the temperature rises slowly to 60°C, the progress of the reaction is tracked by HPLC, and the reaction is complete after 24h. 20 mL of water is added to the reaction solution, the pH value is adjusted to 2~3 with 2N dilute hydrochloric acid, the liquids are separated, the organic phase is discarded. The pH value of the water phase is adjusted to about 4.5with 2N sodium hydroxide solution, a solid is precipitated, filtered to obtain a white crystal (2.60g, 0.0157mol), which is the target product compound 15, with a yield of 78.5%; ee=6.2%. MS(ESI): m/z 166.0787[M+H]⁺.

Example 16:

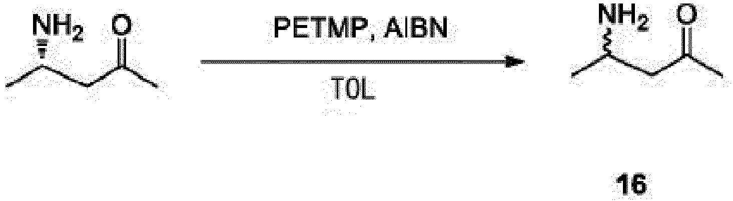

(S)-4-amino-2-pentanone (2.02g, 0.020mol, ee>99%), dry TOL (20ml), pentaerythritol tetra-3-mercaptopropionate (PETMP, 98mg, 0.20mmol), azobisisobutyronitrile (AIBN, 33mg, 0.20mmol) are put into a 100 ml of three-necked flask, magnetic stirring is turned on. Under nitrogen protection, the temperature rises slowly to 80°C, the progress of the reaction is tracked by HPLC, and the reaction is complete after 26h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (1.87g, 0.0185mol), which is the target product compound 16, with a yield of 92.6%; ee=1.8%. MS(ESI): m/z 102.0847[M+H]⁺.

Example 17:

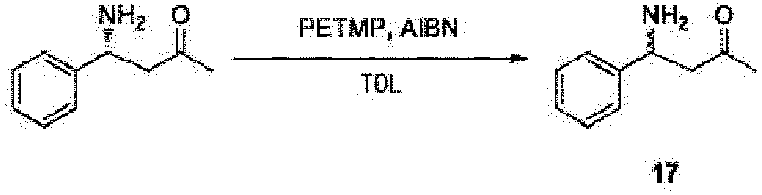

(R)-4-amino-4-phenyl-2-butanone (3.26g, 0.020mol, ee>99%), dry TOL (20ml), pentaerythritol tetra-3-mercaptopropionate (PETMP, 98mg, 0.20mmol), azobisisobutyronitrile (AIBN, 33mg, 0.20mmol) are put into a 100 ml of three-necked flask, magnetic stirring is turned on. Under nitrogen protection, the temperature rises slowly to 100°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (3.17g, 0.0194mol), which is the target product compound 17, with a yield of 97.2%; ee=1.5%. MS(ESI): m/z 164.0995[M+H]⁺.

Example 18:

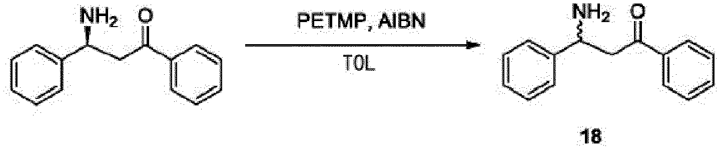

(S)-3-amino-1,3-diphenyl-1-one (4.51g, 0.020mol, ee>99%), toluene (20ml), pentaerythritol tetra-3-mercaptopropionate (PETMP, 98mg, 0.20mmol), azobisisobutyronitrile (AIBN, 33mg, 0.20mmol) are put into a 100 ml of three-necked flask, magnetic stirring is turned on. Under nitrogen protection, the temperature rises slowly to 120°C, the progress of the reaction is tracked by HPLC, and the reaction is complete after 18h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (4.41g, 0.0196mol), which is the target product compound 18, with a yield of 97.8%; ee=1.3%. MS(ESI): m/z 226.1157[M+H]⁺.

Example 19:

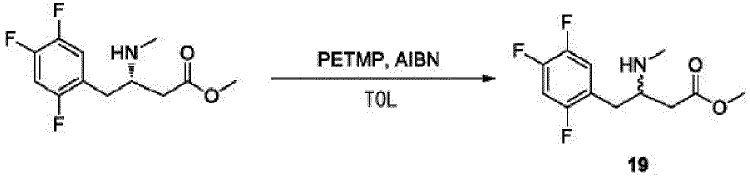

(S)-3-methylamino-4-(2,4,5-trifluorophenyl) butyric acid methyl ester (5.22g, 0.020mol, ee>99%), pentaerythritol tetra-3-mercaptopropionate (PETMP, 0.30g, 0.60mmol), azobisisobutyronitrile (AIBN, 66mg, 0.40mmol) are put into a 100 ml of three-necked flask, magnetic stirring is turned on. Under nitrogen protection, the temperature rises slowly to 80°C, the progress of the reaction is tracked by HPLC, and the reaction is complete after 20h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (4.87g, 0.0187mol), which is the target product compound 19, with a yield of 93.3%; ee=4.8%. MS(ESI): m/z 262.0973[M+H]⁺.

Example 20:

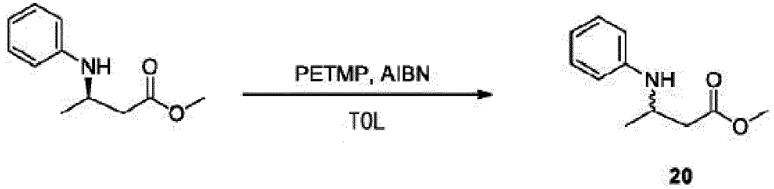

(R)-3-amino-butyric acid methyl ester (3.87g, 0.020mol, ee>99%), toluene (20ml), pentaerythritol tetra-3-mercaptopropionate (PETMP, 0.49g, 0.010mol), azodiisobutyronitrile (AIBN, 66mg, 0.40mmol) are put into a 100 ml of three-necked flask, magnetic stirring is turned on. Under nitrogen protection, the temperature rises slowly to 100°C, the progress of the reaction is tracked by HPLC, and the reaction is complete after 22h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phases is combined, washed once with saturated brine (20ml), dried with anhydrous sodium sulfate (10g), filtered, and the filtrate is concentrated to dryness under reduced pressure at 35~40°C to obtain a colorless oil (3.54g, 0.0183mol), which is the target product compound 20, with a yield of 91.5%; ee=9.7%. MS(ESI): m/z 194.1108[M+H]⁺.

Example 21:

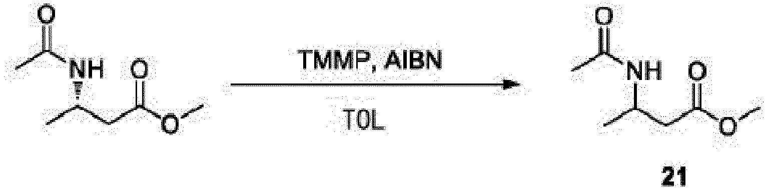

(S)-3-methyl acetaminobutyrate (3.18g, 0.020mol, ee>99%), toluene (20ml), pentaerythritol tetra-3-mercaptopropionate (PETMP, 0.98g, 0.002mol), azodiisobutyronitrile (AIBN, 0.17g, 0.002mol) are put into a 100 ml of three-necked flask, magnetic stirring is turned on. Under nitrogen protection, the temperature rises slowly to 120°C, the progress of the reaction is tracked by HPLC, and the reaction is complete after 24h. 20 mL of water is added to the reaction solution, the pH value is adjusted to 4~5 with 2N dilute hydrochloric acid, the liquids are separated, the organic phase is discarded. The pH value of the water phase is adjusted to 9~10 with 2N sodium hydroxide solution, and extracting is performed twice with isopropyl acetate (2 × 20ml). The organic phase is concentrated to dryness under reduced pressure, and separated by fast column chromatography to obtain a colorless oil (2.46g, 0.0155mol), which is the target product compound 21, with a yield of 77.3%; ee=16.5%. MS(ESI): m/z 160.0897[M+H]⁺.

## Claims

1. A method for a racemic preparation of chiral β -amino acids and derivatives thereof, comprising the steps of:
racemizing a compound of formula I under conditions of initiator, sulfide and solvent to obtain a compound of formula II, wherein a reaction equation is: 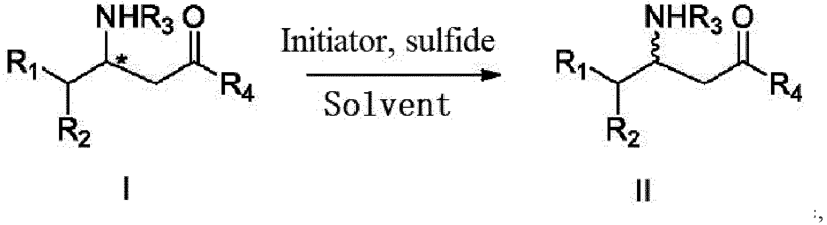 where R₁ or R₂ is hydrogen, alkyl or aryl; R₃ is hydrogen, alkyl, aryl or acyl; R₄ is alkyl, aryl, OR', SR', NHR' or NR'R", where R' or R" is hydrogen, alkyl or aryl.

2. A method for a racemic preparation of chiral β -amino acids and derivatives thereof, comprising the steps of:
racemizing a compound of formula I' under conditions of initiator, sulfide and solvent to obtain a compound of formula II, wherein a reaction equation is: 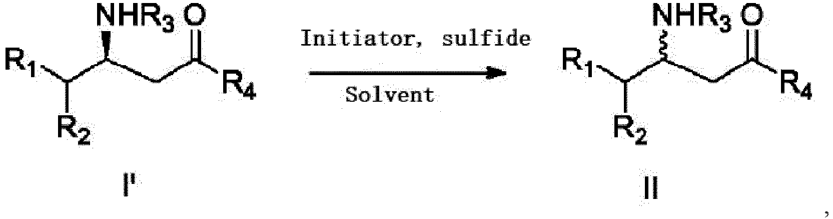 where definitions of R₁, R₂, R₃, R₄ are the same as that according to claim 1.

3. A method for a racemic preparation of chiral β -amino acids and derivatives thereof, comprising the steps of:
racemizing a compound of formula I" under conditions of initiator, sulfide and solvent to obtain a compound of formula II, wherein a reaction equation is: 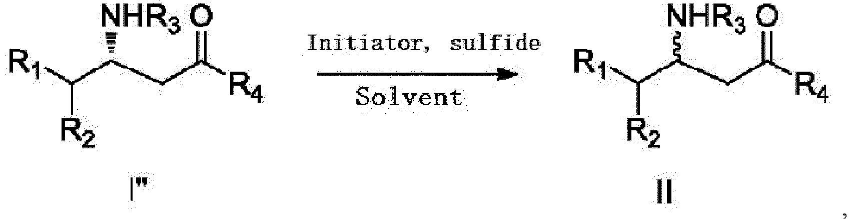 where definitions of R₁, R₂, R₃, R₄ are the same as that according to claim 1.

4. A method for racemizing ethyl 3-amino-4-(2,4,5-trifluorophenyl) butyrate, obtained by racemizing (S)-3-amino-4-(2,4,5-ethyl trifluorophenyl) butyrate under conditions of pentaerythritol tetra-3-mercaptopropionate (PETMP), azobisisobutyronitrile (AIBN) and toluene; wherein a reaction equation is:

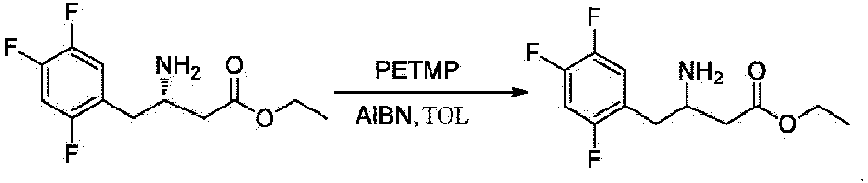

5. The method according to claim 1 or 2 or 3, wherein the sulfide is selected from a group consisting of methyl mercaptoacetate, methyl mercaptopropionate, methyl mercaptobutyrate, ethyl mercaptoacetate, 1-hexyl mercaptan, 1-heptane mercaptan, 1-octyl mercaptan, 1-nonane mercaptan, 1-decane mercaptan, benzyl mercaptan, phenyl ethyl mercaptan, furfuryl mercaptan, dodecane mercaptan and other alkyl mercaptans; 1,6-hexanedithiol, 3-propanedithiol, dimercaptoethyl sulfide, pentaerythritol tetra-3-mercaptopropionate (PETMP), trimethylolpropane tris(3-mercaptopropionate) (TMMP) and other polythiols; mercaptopropyltrimethoxysilane, 3-mercaptopropyltriethoxysilane and other mercapto-containing compounds; dialkyl disulfide, thiophenol, substituted thiophenol and various aromatic thiophenol, diphenyl disulfide and diaryl disulfide.

6. The method according to claim 1 or 2 or 3, wherein the solvent is selected one or more from a group consisting of benzene, toluene, o-xylene, m-xylene, p-xylene, nitrobenzene, chlorobenzene, tetrahydrofuran, 2-methyltetrahydrofuran, 1,4-dioxane, anisole, acetonitrile, chloroform, carbon tetrachloride, n-heptane, and n-octane.

7. The method according to claim 1 or 2 or 3, wherein the initiator is selected from a group consisting of azobisisobutyronitrile (AIBN), azobisisovaleronitrile (AMBN), azobisisoheptonitrile (ABVN), azobisisobutamidine hydrochloride (AIBA), dimethyl azobisisobutyrate (V601, AIBME), azobisisobuimidazoline hydrochloride (AIBI), azo isobutylcyanide formamide and other azo initiator (V30); dibenzoyl peroxide (BPO), lauryl peroxide (LPO), Diisopropyl peroxydicarbonate (IPP), dicyclohexyl peroxydicarbonate (DCPD), di-tert-butyl peroxide (DTB), m-chloroperoxybenzoic acid (m-CPBA), potassium persulfate (K₂S₂O₈) and other peroxide initiators.

8. The method according to claim 1 or 2 or 3 or 4, wherein a reaction temperature is 30°C to 120°C.

9. The method according to claim 1 or 2 or 3 or 4, wherein a reaction time is 4 to 36 hours.

10. The method according to claim 1 or 2 or 3 or 4, wherein a molar ratio of the initiator, the sulfide and a chiral β -amino substrate required for the reaction is that: initiator: sulfide: substrate is (0.01~0.20):(0.01~0.20):1.
